(19) ...

Europäisches
Patentamt

European
Patent Office

Office européen
des brevets

(11) **EP 2 204 416 A1**

(12) **EUROPEAN PATENT APPLICATION**
published in accordance with Art. 153(4) EPC

(43) Date of publication:
**07.07.2010 Bulletin 2010/27**

(51) Int Cl.:
***C08L 53/00*** *(2006.01)*  ***A61L 29/00*** *(2006.01)*
***C08L 75/04*** *(2006.01)*

(21) Application number: **08842671.3**

(22) Date of filing: **27.10.2008**

(86) International application number:
**PCT/JP2008/069425**

(87) International publication number:
**WO 2009/054524 (30.04.2009 Gazette 2009/18)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **27.10.2007 JP 2007279720**

(71) Applicant: **Kaneka Corporation
Kita-ku
Osaka-shi, Osaka 530-8288 (JP)**

(72) Inventors:
• **OHARA, Kazumasa
Settsu-shi
Osaka 566-0072 (JP)**
• **MIYAUCHI, Hidekazu
Settsu-shi
Osaka 566-0072 (JP)**

(74) Representative: **Gille Hrabal Struck Neidlein Prop
Roos
Patentanwälte
Brucknerstrasse 20
40593 Düsseldorf (DE)**

(54) **RESIN COMPOSITION FOR BALLOON AND BALLOON MADE OF THE SAME**

(57) Disposed is a resin composition for balloons, which is excellent in gas permeation resistance and hydrolysis resistance (acid resistance), while having an excellent balance among flexibility, mechanical strength and elastic recoverability. Also disclosed is a balloon made of such a resin composition for balloons. Specifically disclosed is a resin composition for balloons, which is **characterized by** containing an isobutylene block copolymer (A) having a polymer block (a) mainly composed of isobutylene and a polymer block (b) mainly composed of an aromatic vinyl monomer. This resin composition for balloons is also **characterized in that** the polymer block (b) is 10-40% by weight of the weight of the isobutylene block copolymer (A).

EP 2 204 416 A1

**Description**

TECHNICAL FIELD

The present invention relates to a resin composition for balloons and a balloon for use in medical and other applications.

BACKGROUND ART

Silicones have been used widely as balloon materials superior in biocompatibility and flexibility in the field of medicine. However, the silicones are known to be smaller in mechanical strength and inferior in gas permeation resistance, hydrolysis resistance (acid or alkali resistance), and thus, there existed a problem in stability, when they are used for example in medical devices. For example when a silicone balloon is used in a medical balloon catheter, which is placed in the body as it is inflated for one day or longer, such a balloon often cause problems such as need for unintended withdrawal or dislocation of the catheter, because the balloon bursts easily and cannot remain in the inflated state. In particular, there are only silicone products commercially available for gastrostomy balloon catheters, and there existed problems in safety and management, because they highly likely bursts because of its low acid resistance when placed in the stomach, and, as they are placed generally over an extended period of time, it is necessary to confirm the inflation state of the balloon every week, because the gas permeation resistance is lower. There was also a problem that the silicones are lower in processability and demand an adhesive for adhesion of silicone parts. For these reasons, there exists a need for a new material replacing silicones. An example of the material replacing silicones is a thermoplastic polyurethane resin. Thermoplastic polyurethane resins are superior in physical properties such as mechanical strength, biocompatibility and abrasion resistance. They are also material superior in processability, as they can be molded easily by common thermoplastic resin-molding methods (Patent Document 1).

Patent Document 1: JP 6-218037A

SUMMARY OF THE INVENTION

Problems to be Solved by the invention

However, thermoplastic polyurethane resins are poorly flexible and, if the flexibility thereof is improved by reduction of their hardness, it is difficult to produce them efficiently, because the reactive hardening is slower, the viscosity higher and the melting point lower in the synthetic phase. In addition, compositions containing a blended plasticizer may cause problems such as bleeding out. Further, they are not superior in hydrolysis resistance (including acid resistance) and gas permeation resistance and thus, not necessarily suitable for use in the medical device application, for example as a balloon or a balloon catheter for treatment of gastrostomy described above.

The object of the present invention is to provide a resin composition for balloons made of a resin replacing the thermoplastic polyurethane resins and having the advantages of the thermoplastic polyurethanes, that gives a balloon having a gas permeation resistance, a hydrolysis resistance (acid resistance) and a mechanical strength better than those of silicones and having a flexibility, gas permeation resistance and elastic recoverability and hydrolysis resistance (acid resistance) better than those of thermoplastic polyurethane resins, and a balloon and a gastrostomy balloon catheter made of the same.

Means to Solve the Problems

The present invention has the following one or more features.

A resin composition for balloons, characterized by containing an isobutylene block copolymer (A) having a polymer block (a) mainly composed of isobutylene and a polymer block (b) mainly composed of an aromatic vinyl monomer, wherein the content of the polymer block (b) is 10 to 40% by weight of the isobutylene block copolymer (A);

The resin composition for balloons, wherein the ratio (A)/(B) of the isobutylene block copolymer (A) to the thermoplastic polyurethane resin (B) is 100/0 to 40/60 by weight;

The resin composition for balloons, wherein the molecular weight of the isobutylene block copolymer (A) is 50000 to 200000;

The resin composition for balloons, wherein the aromatic vinyl monomer is at least one monomer selected from the group consisting of styrene, p-methylstyrene and $\alpha$-methylstyrene;

The resin composition for balloons, wherein the isobutylene block copolymer (A) is a triblock copolymer having a polymer block mainly composed of an aromatic vinyl monomer, a polymer block mainly composed of isobutylene and a polymer block mainly composed of an aromatic vinyl monomer;

The resin composition for balloons, characterized by containing additionally a polymer (C) containing an olefinic polymer having at least one functional group selected from the group consisting of epoxy group, amino group, hydroxyl group, acid anhydride group, carboxyl group and the salts and carboxylic esters thereof or a styrenic polymer in an amount of 1 to 20 wt parts with respect to 100 wt parts of the total amount of (A) and (B);

The resin composition for balloons, characterized by containing additionally a lubricant (D) in an amount of 0.1 to 10 wt parts or a processing aid (E) in an amount of 0.1 to 10 wt parts with respect to 100 wt parts of the total amount of the isobutylene block copolymer (A) and the thermoplastic polyurethane resin (B);

A balloon or gastrostomy balloon catheter, characterized by being made of the resin composition for balloons;

A gastrostomy balloon catheter, characterized by having a balloon made of the resin composition for balloons in the distal region of a shaft having a lumen inside for flow of fluid for balloon inflation; and

The gastrostomy balloon catheter, wherein the water vapor permeance of the balloon is $1.06 \times 10^{-11}$ or less.

## ADVANTAGEOUS EFFECT OF THE INVENTION

The resin composition for balloons according to the present invention is superior in flexibility, gas permeation resistance, mechanical strength, elastic recoverability and hydrolysis resistance. Thus, it can be used in various fields including general industrial fields, automobile field, medical and pharmaceutical field, food field and others. In particular, it can be used favorably as a balloon having a soft resin layer demanding favorable flexibility, gas permeation resistance, mechanical strength and hydrolysis resistance, in particular as a medical balloon. It can additionally be used favorably as a medical balloon that is placed as it is inflated in body for one day or longer. It can be used particularly favorably as a gastrostomy balloon catheter.

## BEST MODE OF CARRYING OUT THE INVENTION

Hereinafter, the resin composition for balloons according to the present invention and the balloon and the medical balloon prepared by using the same will be described.

1. Isobutylene block copolymer (A)

The isobutylene block copolymer (A) for use in the resin composition for balloons according to the present invention contains a polymer block (a) mainly composed of isobutylene and a polymer block (b) mainly composed of a monomer component other than isobutylene. The term "mainly composed of," as used herein, means that the monomer constitutes the polymer block at least in an amount of 40% or more by weight. However, the monomer content is preferably 60% or more, more preferably 80% or more, when the properties of the resin composition for balloons are considered.

Various monomers can be used as the monomer components other than isobutylene, but the monomer component is preferably a cationic polymerizable monomer component, from the point of synthetic efficiency of the isobutylene block copolymer (A), and examples thereof include monomers such as aliphatic olefins, aromatic vinyls, dienes, vinyl ethers, silanes, vinyl carbazole, β-pinene and acenaphthylene. These monomers may be used alone or in combination of two or more.

Aliphatic olefins (hereinafter, referred to as aliphatic olefin monomers) include ethylene, propylene, 1-butene, 2-methyl-1-butene, 3-methyl-1-butene, pentene, hexene, cylcohexene, 4-methyl-1-pentene, vinylcyclohexane, octene, norbornene and the like.

Aromatic vinyls (hereinafter, referred to as aromatic vinyl monomers) include styrene, o-, m- or p-methylstyrene, α-methylstyrene, 6-methylstyrene, 2,6-dimethylstyrene, 2,4-dimethylstyrene, a-methyl-o-methylstyrene, α-methyl-m-methylstyrene, α-methyl-p-methylstyrene, β-methyl-o-methylstyrene, β-methyl-m-methylstyrene, β-methyl-p-methylstyrene, 2,4,6-trimethylstyrene, α-methyl-2,6-dimethylstyrene, α-methyl-2,4-dimethylstyrene, β-methyl-2,6-dimethylstyrene, β-methyl-2,4-dimethylstyrene, o-, m- or p-chlorostyrene, 2,6-dichlorostyrene, 2,4-dichlorostyrene, α-chloro-o-chlorostyrene, α-chloro-m-chlorostyrene, α-chloro-p-chlorostyrene, β-chloro-o- chlorostyrene, β-chloro-m-chlorostyrene, β-chloro-p-chlorostyrene, 2,4,6-triclolorostyrene, α-chloro-2,6-dichlorostyrene, α-chloro-2,4-dichlorostyrene, β-chloro-2,6-dichlor ostyrene, β-chloro-2,4-dichlorostyrene, o-, m- or p-t-butylstyrene, o-, m- or p-methoxystyrene, o-, m- or p-chloromethylstyrene, o-, m- or p-bromomethylstyrene, silyl-substituted styrene derivatives, indene (aromatic vinyls or aromatic vinyl monomers, when used in the present invention, include indenes), vinylnaphthalene and the like.

Dienes (hereinafter, referred to as diene monomers) include butadiene, isoprene, hexadiene, cyclopentadiene, cyclohexadiene, dicyclopentadiene, divinylbenzene, ethylidene norbornene and the like.

Vinyl ethers (hereinafter, referred to as vinyl ether monomers) include methylvinylether, ethylvinylether, (n- and iso-)propylvinylether, (n-, sec-, tert- and iso-)butylvinylether, methylpropenylether, ethylpropenylether and the like.

Silanes (hereinafter, referred to as silane compounds) include vinyltrichlorosilane, vinylmethyldichlorosilane, vinyldimethylchlorosilane, vinyldimethylmethoxysilane, vinyltrimethylsilane, divinyldichlorosilane, divinyldimethoxysilane, divinyldimethylsilane, 1,3-divinyl-1,1,3,3-tetramethyldisiloxane, trivinylmethylsilane, γ-methacryloyloxypropyltrimethoxysilane, γ-methacryloyloxypropylmethyldimethoxysilane and the like.

The polymer block (b) mainly composed of a monomer component other than isobutylene preferably contains an aromatic vinyl monomer as the principal component, from the point of the balance for example between physical properties and polymerization efficiency. The aromatic vinyl monomer for use is preferably one or more monomers selected from the group consisting of styrene, α-methylstyrene, p-methylstyrene and indene, and use of styrene, α-methylstyrene or the mixture thereof is particularly preferable from the point of cost. In addition, the polymer block (b) preferably contains an aromatic vinyl monomer in an amount of 60 wt % or more, more preferably 80 wt % or more, from the viewpoint of mechanical strength.

On the other hand, the polymer block (a) mainly composed of isobutylene may or may not contain a monomer other than isobutylene, but preferably contains isobutylene in an amount of 60 wt % or more, more preferably 80 wt % or more. The monomer other than isobutylene is preferably a cationic polymerizable monomer such as the monomer described above.

The isobutylene block copolymer (A) is not particularly limited, if it is a compound having a polymer block (a) mainly composed of isobutylene and a polymer block (b) mainly composed of a monomer component other than isobutylene, and may be, for example, a block, diblock, triblock or multi-block copolymer having a straight-chain, branched, star-shaped or other structure. Block copolymers favorable from the point of the balance for example between physical properties and polymerization efficiency include triblock copolymers having a polymer block mainly composed of an aromatic vinyl monomer, a polymer block mainly composed of isobutylene and a polymer block mainly composed of an aromatic vinyl monomer; diblock copolymers having a polymer block mainly composed of an aromatic vinyl monomer and a polymer block mainly composed of isobutylene; star-shaped block copolymers having three arms consisting of a polymer block mainly composed of an aromatic vinyl monomer and a polymer block mainly composed of isobutylene, and the like. These block copolymers may be used alone or in combination of two or more, to obtain favorable physical properties and molding processability.

The rate of the polymer block (a) to the polymer block (b) is not particularly limited, but the content of the polymer block (b) is preferably 5 to 60 wt % and the polymer block (a) 95 to 40 wt %, from the point of balance in physical properties; the content of the polymer block (b) is more preferably 10 to 40 wt % and that of the polymer block (a), 90 to 60 wt %; and the content of the polymer block (b) is particularly preferably 15 to 30 wt % and that of the polymer block (a), 85 to 70 wt %. A content of the polymer block (b) lower than the range above unfavorably leads to insufficient expression of mechanical properties, while a content higher than the range above to deterioration in the isobutylene-derived gas permeation resistance (gas barrier property).

The molecular weight of the isobutylene block copolymer (A) is also not particularly limited, but the number-average molecular weight thereof is preferably 30000 to 500000, particularly preferably 50000 to 200000, from the points of flowability, processability, physical properties and others. A number-average molecular weight of the isobutylene block copolymer lower than the range above disadvantageously leads to deterioration in tackiness (tacky feeling) and bleeding out of its softening agent and also to insufficient expression of favorable mechanical properties, while a molecular weight higher than the range above is disadvantageous from the points of flowability and processability.

The method of producing the isobutylene block copolymer (A) is not particularly limited, but it is prepared, for example, by polymerizing a monomer component mainly composed of isobutylene and a monomer component mainly composed of a monomer other than isobutylene in the presence of a compound represented by the following General Formula (1):

$$(CR^1R^2X)nR^3 \qquad (1)$$

[wherein, X represents a substituent group selected from halogen atoms and alkoxy and acyloxy groups having 1 to 6 carbon atoms; $R^1$ and $R^2$ each represent a hydrogen atom or a monovalent hydrocarbon group having 1 to 6 carbon atoms, and $R^1$ and $R^2$ may be the same as or different from each other; $R^3$ represents a polyvalent aromatic or aliphatic hydrocarbon group; and n is a natural number of 1 to 6.]

The compound represented by the General Formula (1) is a compound playing a role as an initiator, and considered to generate carbon cations in the presence of Lewis acid for example, initiating cationic polymerization. Examples of the compounds of General Formula (1) for use in the present invention include (1-chloro-1-methylethyl)benzene [$C_6H_5C(CH_3)_2Cl$], 1,4-bis(1-chloro-1-methylethyl)benzene [$1,4-Cl(CH_3)_2CC_6H_4C(CH_3)_2$ Cl], 1,3-bis(1-chloro-1-methylethyl) benzene [$1,3-Cl(CH_3)_2CC_6H_4C(CH_3)_2Cl$], 1,3,5-tris(1-chloro-1-methylethyl)benzene [$1,3,5-(ClC(CH_3)_2)_3C_6H_3$], 1,3-bis (1-chloro-1-methylethyl)-5-(tert-butyl)benzene [$1,3-(C(CH_3)_2Cl)_2$ $-5-(C(CH_3)_3)C_6H_3$] and the like.

Particularly preferable among them are bis(1-chloro-1-methylethyl)benzene [$C_6H_4(C(CH_3)_2Cl)_2$] and tris(1-chloro-1-methylethyl)benzene [$(ClC(CH_3)_2)_3C_6H_3$] (bis(1-chloro-1-methylethyl)benzene is also called bis(α-chloroisopropyl)benzene, bis(2-chloro-2-propyl)benzene or dicumyl chloride; and tris(1-chloro-1-methylethyl)benzene is also called tris(α-chloroisopropyl)benzene or tris(2-chloro-2-propyl)benzene or tricumyl chloride).

A Lewis acid catalyst may be present additionally in preparation of the isobutylene block copolymer (A) by polymerization. The Lewis acid is preferably a Lewis acid that can be used in cationic polymerization, and examples of the Lewis acids favorably used include metal halides such as $TiCl_4$, $TiBr_4$, $BCl_3$, $BF_3$, $BF_3$-$OEt_2$, $SnCl_4$, $SbCl_5$, $SbF_5$, $WCl_6$, $TaCl_5$, $VCl_5$, $FeCl_3$, $ZnBr_2$, $AlCl_3$ and $AlBr_3$; and organic metal halides such as $Et_2AlCl$ and $EtAlCl_2$. In particular, $TiCl_4$, $BCl_3$ and $SnCl_4$ are favorable, when the activity and the commercial availability of the catalyst are taken into consideration. The amount of the Lewis acid used is not particularly limited, and determined for example according to the desired polymerization efficiency or the polymerization concentration of the monomers used. It is normally used in an amount in the range of 0.1 to 100 mole equivalences, preferably 1 to 50 mole equivalences, with respect to the compound represented by General Formula (1).

An electron donor component may be present additionally as needed in production of the isobutylene block copolymer (A) by polymerization. The electron donor component is considered to stabilize growing carbon captions during cationic polymerization and addition of the electron donor component makes it easier to produce a polymer narrower in molecular weight distribution. The electron donor component that may be used is not particularly limited, and examples thereof include pyridines, amines, amides, sulfoxides, esters, or metal compounds having oxygen atoms bound to the metal atom, and the like.

Production of the isobutylene block copolymer (A) by polymerization can generally be carried out in an organic solvent, and in particular, it is preferably carried out in an organic solvent that does not essentially inhibit the cationic polymerization. Typical examples thereof include halogenated hydrocarbons such as methyl chloride, dichloromethane, chloroform, ethyl chloride, dichloroethane, n-propyl chloride, n-butyl chloride and chlorobenzene; alkylbenzenes such as benzene, toluene, xylene, ethylbenzene, propylbenzene and butylbenzene; straight-chain aliphatic hydrocarbons such as ethane, propane, butane, pentane, hexane, heptane, octane, nonane and decane; branched aliphatic hydrocarbons such as 2-methylpropane, 2-methylbutane, 2,3,3-trimethylpentane and 2,2,5-trimethylhexane; cyclic aliphatic hydrocarbons such as cyclohexane, methylcyclohexane and ethylcyclohexane; paraffin oils prepared by hydrogenation and purification of petroleum distillates; and the like.

These solvents may be used alone or in combination of two or more, while the balance of the polymerization efficiency of the monomers constituting the block copolymer and the solubility of the polymer obtained, for example, are taken into consideration.

The amount of the solvent used is preferably adjusted to a polymer concentration of 1 to 50 wt %, preferably 5 to 35 wt %, while the viscosity of the obtained polymer solution and the efficiency of heat dissipation are considered.

In actual polymerization, respective components are mixed with each other, as the mixture is cooled, for example, at a temperature of -100°C or higher and lower than 0°C. The polymerization is particularly preferably carried out at a temperature of -30°C to -80°C, when the energy cost and polymerization stability are considered.

2. Thermoplastic polyurethane resin (B)

In the present invention, a thermoplastic polyurethane resin (B) may be mixed arbitrarily with the essential component isobutylene block copolymer (A). In such a case, it is possible to improve the adhesiveness and heat welding efficiency of the resin composition for balloons, as compared to the case where the resin composition for balloons is prepared only with the isobutylene block copolymer (A). Examples of the thermoplastic polyurethane resins (B) for use include various thermoplastic urethane resins such as ester-based, ether-based and carbonate-based polyurethane resins. An example of the thermoplastic polyurethane resin (B) is a thermoplastic polyurethane resin composed of (i) an organic diisocyanate, (ii) a chain extender and (iii) a polymeric polyol. The thermoplastic polyurethane resin (B) may be prepared by any method and, for example, it may be prepared by a known method, for example by mixing the component (i) with a mixture of the components (ii) and (iii) previously prepared under agitation at high speed, pouring the mixture into a vat previously release-finished, and as needed allowing reaction at a temperature of 200°C or lower, or by preparing a terminal isocyanate prepolymer in reaction of the components (i) and (ii), adding the component (iii) thereto and agitating the mixture under high-speed agitation, pouring the mixture into a vat previously release finished, and as needed allowing reaction at a temperature of 200°C or lower.

The organic diisocyanate (i) for use may be a known compound that is selected properly, and examples thereof include hexamethylene diisocyanate, lysine diisocyanate, isophorone diisocyanate, xylene diisocyanate, cyclohexane diisocyanate, toluidine diisocyanate, 2,4-tolylene diisocyanate, 2,6-tolylene diisocyanate, 4,4'-diphenylmethane diisocyanate, p-phenylene diisocyanate, m-phenylene diisocyanate, 1,5-naphthalene diisocyanate and the like. These diisocyanates may be used alone or in combination of two or more.

The chain extender (ii) for use is preferably a dihydroxy compound having a molecular weight of less than 500. Examples thereof include ethylene glycol, 1,2-propylene glycol, 1,3-propylene glycol, 2,3-butylene glycol, 1,4-butanediol, 2,2'-dimethyl-1,3-propanediol, diethylene glycol, 1,5-pentanediol, 1,6-hexanediol, cyclohexane-1,4-diol, cyclohexane-1,4-dimethanol and the like, and these compounds may be used alone or in combination of two or more.

The polymeric polyol (iii) for use is preferably a dihydroxy compound having an average molecular weight of 500 to 4000. Examples thereof include polyester diols, polyether diols, polycarbonate diols and the like. Examples of the polyester diols include polycondensates of one or more low-molecular weight diol components selected from ethylene glycol, propylene glycol, 1,4-butanediol, 1,5-pentanediol, 1,6-hexanediol, neopentylglycol, 3-methyl-1,5-pentanediol, cyclohexanedimethanol and others with one or more low-molecular weight dicarboxylic acids selected from glutaric acid, suberic acid, sebacic acid, terephthalic acid, isophthalic acid and others; and polylactone diols obtained by ring-opening polymerization of lactones such as polypropiolactone diol, polycaprolactone diol and polyvalerolactone diol; and the like. Examples of the polyether diols include polypropylene ether glycol, polytetramethylene ether glycol, polyhexamethylene ether glycol, other copolymerization polyether glycols and the like. Examples of the polycarbonate diols include polyhexamethylene carbonate diol; diols obtained by ring-opening addition polymerization of a lactone to polyhexamethylene carbonate diol; co-condensates of polyhexamethylene carbonate diol with other polyester diols, polyether diols, polyether diols or polyester diols; and the like.

On the other hand, the ratio (A)/(B) of the isobutylene block copolymer (A) to the thermoplastic polyurethane resin (B) is preferably 100/0 to 10/90, more preferably, 100/0 to 40/60 by weight. A component (A) rate of less than 10 wt % may lead to insufficient improvement in flexibility and gas permeation resistance.

3. Polymer (C)

In the present invention, when an isobutylene block copolymer (A) is mixed with a thermoplastic polyurethane resin (B), a polymer (C) may be added additionally for improvement in compatibility between (A) and (B). Examples of the polymers (C) include olefinic and styrenic polymers having at least one functional group selected from the group consisting of epoxy, amino, hydroxyl, acid anhydride, carboxylic acid and the salts and carboxylic esters thereof. The polymer (C) may be a copolymer, and the copolymerization form of the copolymer is not particularly limited, and the copolymer may be, for example, a random copolymer, a graft copolymer, a block copolymer or the like.

Examples of the olefinic and styrenic polymers carrying the functional group for the polymer (C) (herein, the method of introducing the functional group is not particularly limited, and the functional group may be introduced, for example, by a method of modifying a polymerized support polymer or a method of mixing and copolymerizing a support-forming monomers) include ethylene-$\alpha$-olefin copolymers such as ethylene-propylene copolymers, ethylene-butene copolymers, ethylene-octene copolymers and ethylene-hexene copolymers; polyethylene, polypropylene, polystyrene, polybutene, ethylene-propylene-diene copolymers, styrene-butadiene copolymers, styrene-butadiene-styrene block copolymers (SBS), styrene-isoprene-styrene block copolymers (SIS), polybutadiene, butadiene-acrylonitrile copolymers, polyisoprene, butene-isoprene copolymers, styrene-ethylene butylene-styrene block copolymers (SEBS), styrene-ethylene propylene-styrene block copolymers (SEPS) and the like.

Typical examples of the polymers (C) having a functional group include copolymers of a polyolefin polymer such as ethylene-$\alpha$-olefin copolymer with an acid anhydride such as maleic anhydride, succinic anhydride or fumaric anhydride; copolymers of an olefinic polymer with a carboxylic acid such as acrylic acid, methacrylic acid, or vinyl acetate or the salt (Na, Zn, K, Ca, Mg or the like) thereof or a carboxylic ester such as methyl acrylate, methyl methacrylate, ethyl acrylate, ethyl methacrylate, propyl acrylate, propyl methacrylate, butyl acrylate, or butyl methacrylate; and the like.

More specific examples thereof include ethylene-methyl acrylate copolymers, ethylene-ethyl acrylate copolymers, ethylene-n-propyl acrylate copolymers, ethylene-isopropyl acrylate copolymers, ethylene-n-butyl acrylate copolymers, ethylene-t-butyl acrylate copolymers, ethylene-isobutyl acrylate copolymers, ethylene-methyl methacrylate copolymers, ethylene-ethyl methacrylate copolymers, ethylene-n-propyl methacrylate copolymers, ethylene-isopropyl methacrylate copolymers, ethylene-n-butyl methacrylate copolymers, ethylene-t-butyl methacrylate copolymers, ethylene-isobutyl methacrylate copolymers, ethylene-(meth)acrylic acid copolymers and the salts (Na, Zn, K, Ca, Mg etc.) thereof, ethylene-maleic anhydride copolymers, ethylene-butene-maleic anhydride copolymers, ethylene-propylene-maleic anhydride copolymers, ethylene-hexene-maleic anhydride copolymers, ethylene-octene-maleic anhydride copolymers, propylene-maleic anhydride copolymers, maleic anhydride-modified SBSs, maleic anhydride-modified SISs, maleic anhydride-modified SEBSs, maleic anhydride-modified SEPSs, maleic anhydride-modified ethylene-ethyl acrylate copolymers and the like. These copolymers may be used alone or in combination of two or more. In particular, acid anhydride group-containing styrene-ethylene butylene-styrene copolymers (maleic anhydride-modified SEBSs) are preferable from the point of transparency.

The resin composition for balloons according to the present invention preferably contains the polymer (C) in an amount of 0.1 to 50 wt parts, more preferably 1 to 20 wt parts, with respect to 100 wt parts of the total amount of the isobutylene block copolymer (A) and the thermoplastic polyurethane resin (B). A polymer (C) content of less than 0.1 wt part may lead to insufficient expression of compatibility, while a content of more than 50 wt parts to decrease in the rate of component (A) in the composition and deterioration in flexibility, gas permeation resistance and low restitution.

The polymer (C) may be added during melt blending of the isobutylene block copolymer (A) and the thermoplastic polyurethane resin (B) or alternatively to one or both of the isobutylene block copolymer (A) and the thermoplastic polyurethane resin (B) previously. It is preferable to add it previously to the isobutylene block copolymer (A) or the thermoplastic polyurethane resin (B), because improvement in compatibility is more distinctive.

4. Lubricant

The resin composition for balloons according to the present invention may contain, as needed, a lubricant as component (D). Examples of the lubricants favorably used include fatty acid metal salt lubricants, fatty acid amide lubricants, fatty acid ester lubricants, fatty acid lubricants, aliphatic alcohol lubricants, partial ester of a fatty acid with a polyvalent alcohol, paraffin lubricants and the like, and two or more of them may be used in combination.

Examples of the fatty acid metal salt lubricants include calcium stearate, magnesium stearate, aluminum stearate, zinc stearate, barium stearate, montanic acid metal salts and the like.

Examples of the fatty acid amide lubricants include ethylene bisstearic amide, erucic amide, oleic amide, stearic amide, behenic amide, ethylenebisoleic amide, ethylenebiserucic amide, ethylenebislauric amide, m-xylylenebisstearic amide, p-phonylenebisstearic amide and the like.

Examples of the fatty acid ester lubricants include methyl laurate, methyl myristate, methyl palmitate, methyl stearate, methyl oleate, methyl erucate, methyl behenate, butyl laurate, butyl stearate, isopropyl myristate, isopropyl palmitate, octyl palmitate, palm fatty acid octyl ester, octyl stearate, special beef tallow fatty acid octyl ester, lauryl laurate, stearyl stearate, behenyl behenate, cetyl myristate, hardened beef tallow oil, hardened castor oil, montanic esters and the like.

Examples of the fatty acid lubricants include stearic acid, palmitic acid, oleic acid, linolic acid, linoleic acid, montanic acid and the like. Examples of the aliphatic alcohol lubricants include stearyl alcohol, cetyl alcohol, myristyl alcohol, lauryl alcohol and the like. Examples of the partial ester of a fatty acid with a polyvalent alcohol include stearic monoglyceride, stearic diglyceride, oleic monoglyceride, partial saponified montanic ester and the like.

Examples of the paraffin lubricants include paraffin waxes, liquid paraffins, polyethylene waxes, oxidized polyethylene waxes, polypropylene waxes and the like. Among them, fatty acid amide lubricants, fatty acid ester lubricants and paraffin lubricants are preferable as the lubricants, from the point of balance between improvement effect of moldability and cost.

The lubricant (D) is preferably added in an amount of 0.1 to 10 wt parts, with respect to 100 wt parts of the total amount of the isobutylene block copolymer (A) and the thermoplastic polyurethane resin (B). An addition amount of less than 0.1 wt part may lead to deterioration in lubricity to metal face and thus, to deterioration in processability and surface smoothness caused by adhesion, while an addition amount of more than 10 wt parts may prohibit sufficiently mixing, leading to separation of the component (D).

5. Processing aid

In addition, the resin composition for balloons according to the present invention may contain a processing aid (E) additionally. The processing aid (E), which is used for improvement of the melt viscosity of composition, improves moldability during molding. Specifically, it is an inorganic processing improver, an acrylic polymer-based processing improver, a polytetrafluoroethylene-based processing improvers or the like, and typical examples of the polytetrafluoroethylene-based improvers include Metablen (registered trade name) A3000 manufactured by Mitsubishi Rayon Co., Ltd. and Lumifron (registered trade name) manufactured by Asahi Glass Co., Ltd. and the like. The blending rate of the processing aid (E) is not particularly limited, but is preferably 0.1 to 10 wt parts with respect to 100 wt parts of the total amount of the isobutylene block copolymer (A) and the thermoplastic polyurethane resin (B). A blending rate of less than 0.1 wt part may lead to insufficient improvement in processability, while a blending rate of more than 10 wt parts to excessive increase in melt tension and unfavorable molding processability.

6. Molding fluidity-improving aid

The resin composition for balloons according to the present invention may contain, as needed, a polyolefin resin for improvement in molding fluidity additionally. One, or a combination of two or more, of α-olefinic homopolymers, random copolymers, block copolymers and the mixtures thereof, and random, block and graft copolymers of an α-olefin and another unsaturated monomer and those modified by oxidation, halogenation or sulfonation may be used as the polyolefin resin. Typical examples thereof include polyethylene resins such as polyethylene, ethylene-propylene copolymer, ethylene-propylene-unconjugated diene copolymers, ethylene-butene copolymers, ethylene-hexene copolymers, ethylene-octene copolymers, ethylene-vinyl acetate copolymers, ethylene-vinyl alcohol copolymers, ethylene-ethyl acrylate copolymers, ethylene-acrylate copolymer, ethylene-methyl acrylate-maleic anhydride copolymers and chlorinated polyethylenes; polypropylene resins such as polypropylene, propylene-ethylene random copolymers, propylene-ethylene block copolymers and chlorinated polypropylenes; polybutene, polyisobutylene, polymethylpentene, cyclic olefin (co) polymers and the like. Polyethylene, polypropylene or the mixture thereof are used favorably among them, from the point of the balance of the cost and the physical properties of the thermoplastic resin. The blending rate of the polyolefin resin is 0 to 100 wt parts, preferably 0 to 50 wt parts, more preferably 0 to 30 wt parts, with respect to 100 wt parts of the total amount of the isobutylene block copolymer (A) and the thermoplastic polyurethane resin (B). A blending rate of more than 100 wt parts may lead to increase in hardness and is thus unfavorable.

7. Softening agent

The resin composition for balloons according to the present invention may contain, as needed, a softening agent additionally. The kind of the softening agent is not particularly limited, but normally, materials that are liquid at room temperature are used favorably. Both hydrophilic and hydrophobic softening agents can be used. The softening agents include various rubber or resin softening agents based on mineral oils, vegetable oils and synthetic substances. Examples of the mineral oils include process oils such as those based on naphthene and paraffins; examples of the vegetable oils include castor oil, cottonseed oil, flaxseed oil, rapeseed oil, soy bean oil, palm oil, peanut oil, Japan tallow, pine oil, olive oil and the like; and examples of synthetic substances include polybutene, low-molecular weight polybutadiene and the like. A paraffinic process oil or polybutene is used favorably among them, from the point of balance of compatibility with the isobutylene block copolymer (A) and physical properties of the thermoplastic resin composition. These softening agents may be used alone or in combination of two or more for obtaining desired viscosity and physical properties.

The blending rate of the softening agent is 0 to 100 wt parts, preferably 0 to 50 wt parts, more preferably 0 to 30 wt parts, with respect to 100 wt parts of the total amount of the isobutylene block copolymer (A) and the thermoplastic polyurethane resin (B). A blending rate of more than 100 wt parts may lead to bleeding out of the softening agent and is thus unfavorable.

8. Filler

In addition, the resin composition for balloons according to the present invention may contain various fillers for improvement in physical properties or because of economical merit. Examples of favorable fillers include clay, diatomaceous earth, silica, talc, barium sulfate, calcium carbonate, magnesium carbonate, metal oxides, mica, graphite, scaly inorganic fillers such as aluminum hydroxide, various metal powders, wood chips, glass powders, ceramics powders, carbon black, granular or powdery solid fillers such as of granular or powdery polymers, other various natural or synthetic short fibers and continuous filaments, and the like. It is also possible to reduce the weight of the composition by using a hollow filler: an inorganic hollow filler such as of glass or silica balloons or an organic hollow filler for example of polyvinylidene fluoride or a polyvinylidene fluoride copolymer. Various blowing agent may be added thereto for further reduction in weight and improvement in various physical properties including shock absorbance, and a gas may be mixed mechanically during mixing.

The blending rate of the filler is 0 to 100 wt parts, preferably 0 to 50 wt parts, more preferably 0 to 30 wt parts, with respect to 100 wt parts of the total amount of the isobutylene block copolymer (A) and the thermoplastic polyurethane resin (B). A blending rate of more than 100 wt parts may lead to deterioration in mechanical strength and also in flexibility of the obtained thermoplastic resin composition and is thus unfavorable.

In addition, the resin composition for balloons according to the present invention may contain, as needed, an antioxidant and/or an ultraviolet absorbent. The blending rate thereof is 0.01 to 10 wt parts, preferably 0.01 to 5 wt parts, with respect to 100 wt parts of the thermoplastic resin. Other additives that may be added additionally include flame retardants, antibacterial agents, photostabilizers, colorants, fluidity improvers, blocking inhibitors, antistatic agents, crosslinking agents, crosslinking aids and the like, and these substances may by used alone or in combination of two or more. Various thermoplastic resins, thermosetting resins, other thermoplastic elastomers and others may be added further in the ranges that do not impair the properties of the thermoplastic resin composition according to the present invention.

9. Method of producing the resin composition for balloons

The method of producing the resin composition for balloons according to the present invention is not particularly limited, and any known method may be applied. The resin composition is produced, for example, by melt-extruding the components and as needed the additive components through a heated kneader, such as uniaxial extruder, biaxial extruder, roll, Banbury mixer, Brabender mixer, kneader, high-shear mixer. The order of mixing the respective components is not particularly limited, and may be determined according to the equipment used, processability or the desired physical properties of the thermoplastic resin composition obtained.

10. Configuration of balloon

The balloon prepared from the resin composition for balloons may be a single-layer balloon of a single material, a multi-layer balloon containing multiple layers of balloon resin compositions different in composition or a multi-layer balloon containing multiple layers of the balloon resin composition and other resin compositions. For example, when the balloon according to the present invention is a surgical balloon (for use outside blood vessel), it may be a single-layer balloon of the resin composition for balloons or a balloon having a layer of the balloon resin composition as the main or central layer.

For example, a coating layer (coat layer), an antistatic layer and also other layers of different resin compositions may be formed on the top and bottom faces of the layer of the balloon resin composition. The coating layer, when formed on the outermost surface of the balloon, exhibits effectively favorable properties of the coating layer such as gas barrier property (oxygen permeation resistance, water vapor permeation resistance or the like), light blocking property, blocking resistance, slipping resistance, γ-ray resistance, heat resistance, polyvinyl chloride-like texture and others, thus further improving the properties of the balloon. In addition, various processings including embossing and various surface treatments such as acid treatment may be applied on the balloon surface (both internal and external surfaces).

11. Method of producing tube for balloon

The tube for balloon can be prepared by various methods including heat-compression molding, injection molding, dipping molding, extrusion molding and others. For example when it is produced by extrusion molding, the resin composition for balloons obtained by the production method described above is fed into an extruder. The resin is then extruded out of a die and water-cooled into a desired tube for balloon. It is possible to obtain a transparent balloon by using an isobutylene block copolymer and a thermoplastic polyurethane resin as principal components and additionally adjusting the amounts of the third components: functional group-containing styrenic polymer, lubricant, processing aid and others. It is possible to obtain various balloons including medical balloons by processing these balloons.

The balloon and the medical balloon according to the present invention, which are made of a mixture (blend) containing an isobutylene block copolymer (A) and a thermoplastic polyurethane resin (B) and, as needed a lubricant, a processing aid and others, are superior in flexibility.
Specifically, they have the abrasion resistance of the thermoplastic polyurethane resin and also the flexibility similar to that of silicone. They also exhibit high elastic layer recoverability and retain their original shapes even after repeated inflation and contraction. The medical balloon has a flexibility (softness) and inflation efficiency satisfying the level needed for medical balloons.

As described above, the balloon composition according to the present invention can be used favorably in application as medical balloons (such as surgical balloon and intracatheter). It is also extremely useful as an alternative for less-flexible PVC materials, thermoplastic polyurethane resins, soft polyvinyl chloride materials, allergenic natural rubber materials, and silicone materials with poor gas-barrier property.

12. Examples of balloon products

The resin composition for balloons is usable in general industrial fields, automobile field, medical and pharmaceutical fields and food field. In particular, it is favorably used as a medical balloon which requires favorable flexibility, gas permeation resistance, mechanical strength and hydrolysis resistance of a soft resin layer. It can be used, for example, as enteral nutrition catheter, bile duct catheter, urinary catheter, bronchial tube, center vein catheter, gastrostomy catheter, suction catheter, angiographic catheter, cardiac catheter, vasodilation catheter, thrombus ablation catheter, epidural catheter, endoscopic catheter, endogastric balloon or the like. It can also be used without inflation as a device in the balloon shape.

The resin composition for balloons according to the present invention is favorable as a balloon material for gastrostomy catheter among the balloon products above. A gastrostomy catheter, i.e., a gastrostomy balloon catheter, penetrates through abdominal and stomach walls into the stomach at the distal region including the balloon; and the distal region including the balloon is placed there in the stomach for a period as long as several months. Thus, the balloon used as a gastrostomy catheter should have strength large enough to withstand the stomach peristaltic movement, elastic recoverability holding the balloon in the same shape during insertion and withdrawal of the gastrostomy catheter, acid resistance (hydrolysis resistance) withstanding stomach acid, and gas or liquid (fluid) permeation resistance allowing placement thereof in the stomach for an extended period of time.
On the other hand, currently commercially available gastrostomy balloon catheters, which are all silicone products, are lower in acid resistance and thus the risk of balloon rupture is higher, and there is a problem in safety and management that it is necessary when the balloon is placed in stomach to examine whether the fluid (mainly distilled water) contained in the balloon is preserved in a suitable amount. In contrast, the gastrostomy catheter according to the present invention is superior in acid resistance and liquid (fluid) permeation resistance, and thus, the possibility of balloon rupture is extremely low and there is no need for monitoring of the residual amount of the fluid in the balloon for several months. For that reason, the gastrostomy balloon catheter according to the present invention is safer and easily controlled.

The gastrostomy catheter according to the present invention for use may have a known structure. Specifically, the balloon may be formed on the distal region of a shaft having a lumen (balloon lumen) for supply of balloon inflation fluid. More specifically, the shaft may have additionally a lumen (nutrition lumen) for injection of nutrition into the stomach externally, an opening communicating with the nutrition lumen for injection of the nutrition on the proximal region of the shaft, and a hub having an opening for injection of the fluid for balloon inflation communicating with the balloon lumen. The opening in the hub for injection of the fluid for balloon inflation preferably has a check valve.

It is also possible to place an extracorporeal fixing device for fixing the gastrostomy catheter. It is possible in this way to hold abdominal and stomach walls between the inflated balloon and the fixing device and thus fix the gastrostomy catheter.

EXAMPLES

First, various test and evaluation methods will be described below.

(1) Tensile strength and elongation (mechanical strength)

According to JIS K-6251 (Determination of tensile stress-strain properties of vulcanized rubber), a press sheet having a thickness of 1.0 mm was prepared with a resin composition for balloons and cut to a dumbbell-shaped test piece No. 7, and the tensile strength and elongation were determined under the condition of 23°C and 500 mm/minute by using the test piece. The tensile elongation was elongation at break, as determined when the gauge length was 10 mm. The analyzer used was Strograph EII (Toyoseiki Co., Ltd.).

(2)Oxygen permeance (gas permeation resistance)

A resin composition for balloons was compression-molded at 190°C, to give a sample having a thickness of approximately 0.5 mm, and the oxygen permeance thereof was determined at 23°C by using the sample according to JIGS K-7126.

(3) Water vapor permeance (gas permeation resistance)

A resin composition for balloons was compression-molded at 190°C, to give a sample having a thickness of approximately 0.5 mm, and the water vapor permeance thereof was determined at 40°C by using the sample according to JIS K-7129.

(4) Acid resistance (hydrolysis resistance)

According to JIS K-6258 (Determination of the effect of liquids on vulcanized rubber), a press sheet having a thickness of 1.0 mm was prepared with a resin composition for balloons and cut into a dumbbell-shaped test piece No. 7, and the test piece was immersed in the artificial gastric juice (as specified in Japanese Pharmacopoeia) at 40°C for 6 weeks. The acid resistance was determined under the condition of 23°C and 500 mm/minute by using the test piece. The instrument used was Strograph EII (Toyoseiki Co., Ltd.).

(5) Constant tensile permanent set (elastic recoverability)

With reference to JIS K-6273 (Determination of tension set, elongation and creep of vulcanized rubber), a press sheet having a thickness of 1.0 mm was prepared with a resin composition for balloons and cut into a dumbbell-shaped test piece No. 7, and the deformation of the test piece after it was expanded to 300% at 23°C under constant tension for 24 hours and then relaxed for 30 minutes was determined by using the test piece.

The number-average molecular weight shown in Preparative Examples is a value, as determined by using a gel-permeation chromatographic (GPC) system manufactured by Waters (column: Shodex K-804 (polystyrene gel) by Showa Denko K.K., mobile phase: chloroform) and polystyrene particles as standards.

(Preparative Example 1)

Preparation of styrene-isobutylene block copolymer 1

452 mL of 1-chlorobutane (previously dried over molecular sieves), 319 mL of hexane (previously dried over molecular sieves) and 0.55 g of 1,4-bis(1-chloro-1-methylethyl)benzene were placed in a 2L reactor equipped with a stirrer. The reactor was cooled to -75°C, and 0.42 g of dimethylacetamide and 182 mL of isobutylene were added thereto. In addition, 6.53 mL of titanium tetrachloride was added thereto for initiation of polymerization, and the mixture was allowed to react while the solution was stirred at -75°C for 1.5 hours. 51 g of styrene was then added to the reaction solution; the mixture was allowed to react additionally for 60 minutes; and the reaction solution was poured into a large amount of water for termination of the reaction.

Separation of the organic and aqueous phases, as examined by visual observation, was favorable, and the organic phases were fractioned easily with a separatory funnel. The organic phase was washed with water twice; after confirmation that the aqueous phase is neutral, the organic layer was poured into a large amount of methanol for precipitation of the polymer, and the polymer obtained was vacuum-dried at 60°C for 24 hours, to give an isobutylene block copolymer (SIBS). The number-average molecular weight of the SIBS obtained was 72000 and the styrene content was 29 wt %.

(Preparative Example 2)

Preparation of styrene-isobutylene block copolymer 2

2166 mL of methylcyclohexane (previously dried over molecular sieves), 1634 mL of methylene chloride (previously dried over molecular sieves) and 1.756 g of p-dicumyl chloride were placed in a 10L reactor having a stirring blade. The reactor was cooled to -70°C, and then, 0.75 mL of $\alpha$-picoline (2-methylpyridine) and 633 mL of isobutylene were added thereto. In addition, 30 mL of titanium tetrachloride was added thereto for initiation of polymerization, and the mixture was allowed to react at -70°C for 1.5 hours while the solution was agitated. 270 mL of styrene was then added to the reaction solution; the mixture was allowed to react additionally for 20 minutes; and the reaction was terminated by addition of a large amount of methanol. After removal of the solvent and others from the reaction solution, the polymer was dissolved in toluene and washed with water twice. The toluene solution was poured into an acetone-methanol mixture for precipitation of the polymer, and the polymer obtained was vacuum-dried at 60°C for 24 hours, to give SIBS. The number-average molecular weight of the SIBS obtained was 110,000 and the styrene content was 15 wt %.

(Examples 1 to 6 and Comparative Examples 1 and 2)

An isobutylene block copolymer (A) and a thermoplastic polyurethane resin (B) were mixed at the rate shown in Table 1, and the mixture was melt-extruded in a biaxial extruder (manufactured by Labtech Engineering Company Ltd.) set to 190°C. Test pieces were then prepared and used for evaluation of various physical properties. As for silicone, a commercially available silicone sheet was cut into test pieces. The evaluation results are summarized in Table 1. Details of the components used were shown below with their abbreviations.

Isobutylene block copolymer (A)

SIBS obtained in Preparative Example 1 (hereinafter, referred to as SIBS1) SIBS obtained in Preparative Example 2 (hereinafter, referred to as SIBS2) Thermoplastic polyurethane resin (B)
Tecoflex EG-85 A manufactured by Noveon (ether-type thermoplastic polyurethane, hardness: 77 (JIS A), hereinafter, referred to as TPU) Silicone
Silicone sheet manufactured by Togawa Rubber Co., Ltd. (hereinafter, referred to as SIR)
[Table 1]

| | | Example 1 | Example 2 | Example 3 | Example 4 | Example 5 | Example 6 | Comparative Example 1 | Comparative Example 2 |
|---|---|---|---|---|---|---|---|---|---|
| Isobutylene block copolymer (A) | SIBS 1 | 100 | 80 | 40 | | | | | |
| | SIBS 2 | | | | 100 | 40 | 20 | | |
| Thermoplastic polyurethane resin (B) | TPU | | 20 | 60 | | 60 | 80 | 100 | |
| Silicone | SIR | | | | | | | | 100 |
| Tensile strength | MPa | 14.1 | 12.3 | 13.9 | 16.4 | 12.2 | 32.2 | 32.8 | 8.2 |
| Tensile elongation | % | 600 | 544 | 552 | 723 | 624 | 676 | 691 | 393 |
| Oxygen permeance | mol·m/m$^2$·s· Pa | $2.97 \times 10^{-16}$ | $4.82 \times 10^{-16}$ | $1.52 \times 10^{-15}$ | $3.24 \times 10^{-16}$ | $1.34 \times 10^{-15}$ | $2.20 \times 10^{-15}$ | $3.09 \times 10^{-15}$ | $2.00 \times 10^{-13}$ |
| Water vapor permeance | g*cm2*s*cmHg | $1.75 \times 10^{-13}$ | $3.59 \times 10^{-13}$ | $7.66 \times 10^{-12}$ | $1.69 \times 10^{-13}$ | $8.94 \times 10^{-12}$ | $1.06 \times 10^{-11}$ | $1.18 \times 10^{-11}$ | $1.89 \times 10^{-11}$ |
| Acid resistance | MPa | 12.7 | 11.5 | 8.5 | 9.6 | 9.5 | 11.9 | 9.9 | 3.7 |
| Constant-tension permanent set | % | 6.1 | 9.2 | 8.3 | 8.1 | 10.3 | 9.7 | 17.9 | 3.6 |

As obvious from comparison of the results in Examples 1 to 6 and those in Comparative Examples 1 and 2, the samples in Examples 1 to 6 are superior in balance of flexibility, mechanical strength (tensile strength), gas permeation resistance (oxygen and water vapor permeabilities) and hydrolysis resistance (acid resistance). In particular, the samples of Examples 1, 2 and 4 were particularly superior.

These favorable physical properties probably derive from the structure of the resin compositions. Specifically, the resin composition of Examples 2, 3, 5 and 6 have a sea-island structure of SIBS and TPU, and the sample is considered to have physical properties reflecting those of the sea component.

For that reason, the compositions according to the present invention are resin compositions for balloons superior in mechanical strength (tensile strength), gas permeation resistance (oxygen and water vapor permeabilities) and hydrolysis resistance (acid resistance).

(Examples 7 to 9 and Comparative Example 3)

(Balloon molding)

A resin composition for balloons was prepared in a manner similar to Examples 1 to 5, by using the components at the rate shown in Table 2. The resin composition for balloons obtained was then supplied to an uniaxial extruder (tube-molding machine) and extruded out of a tube die connected to the terminal, to give a single-layer balloon tube having an external diameter of 4.7 mm and an internal diameter of 4.2 mm. The inflation efficiency and the load demanded when the tube obtained is used as a medical balloon were evaluated. Evaluation results are summarized in Table 2. The details and abbreviations of the components above are the same as those described above.

Alternatively in Comparative Example 3, a single-layer balloon tube having an external diameter of 4.7 mm and an internal diameter of 4.2 mm was prepared under the condition identical with that of Examples 7 to 9 by using the resin identical with that in Comparative Example 1, and the inflation efficiency and the load were tested similarly, but the balloon was inflated not spherically, but only locally, prohibiting data acquisition (indicated by "n.d." in Table 2).

The physical properties of the balloon tube are determined by the methods shown below.

(Inflation efficiency and internal pressure)

A hole 2 was formed in a polytetrafluoroethylene tube 1 having an external diameter of 4.2mm in the direction vertical to the tube longitudinal direction (used later as an injection port of the medium for inflating the balloon tube), and a balloon tube 3 was placed thereon covering the hole, and both terminals of the tube were fixed with shrink tubes 4. The distance of the fixing shrink tubes was 10 mm. In addition, one terminal 5 of the polytetrafluoroethylene tube 1 was then closed, and water was injected through the other terminal 6 with a syringe, and the inflation state of the balloon was observed. A pressure meter (Pressure Transducer, model VHR3-A3-500Kpa H-5, manufactured by VALCOM) was connected to the site between the polytetrafluoroethylene tube 1 and the syringe. When the maximum amount of water was injected, the balloon internal pressure was measured by the pressure meter and the balloon external diameter was determined with a micrometer (manufactured by KEYENCE Corporation: LS-7600).

[Table 2]

| | | Example 7 | Example 8 | Example 9 | Comparative Example 3 |
|---|---|---|---|---|---|
| Isobutylene block copolymer (A) | SIB1 | 100 | 80 | | |
| | S1B2 | | | 100 | |
| Thermoplastic polyurethane resin (B) | TPU | | 20 | | 100 |
| Internal pressure at break | kPa | 52 | 47 | 19 | n.d. |
| Maximum inflation rate | % | 500 | 490 | 680 | n.d. |

All samples (Examples 7 to 9) were inflated in an almost spherical balloon shape. The internal pressure at break was small and the balloon was inflated easily in each case. There is difference in the balance of inflation rate and internal pressure between Example 7 (SIBS1) and Example 9 (SIBS2), and it is possible to provide balloons with wider varieties of physical properties by blending urethane and other components thereto. On the other hand, the balloon of Comparative Example 3 was found to be unsatisfactory as a balloon material.

(Example 10 and Comparative Example 4)

(Balloon molding)

The resin composition of Example 1 was supplied to an uniaxial extruder (tube-molding machine) and extruded out of a tube die connected to the terminal of the extruder, to give a single-layer balloon tube having an external diameter of 6.9 mm and an internal diameter of 7.7 mm. The balloon tube obtained (Example 10) was compared with commercially available silicone gastrostomy catheter balloon tube (Comparative Example 4). Evaluation results on liquid (water vapor) permeation resistance are summarized in Table 3 and evaluation results on acid resistance are summarized in Table 4.

Physical properties of the balloon tubes are evaluated by the methods shown below.

(Liquid (water vapor) permeation resistance)

A hole was formed in the inflation lumen of a double lumen tube having an external diameter of 6.7 mm in the direction vertical to the tube longitudinal direction, (used later as an injection port of the medium for inflating the tube for balloon), and a balloon tube was placed thereon covering the hole, and both terminals thereof were fixed with shrink tubes. The fixing distance was 20 mm. In addition, one terminal of the inflation lumen was closed; water was injected through the other terminal with a syringe to a balloon diameter of 27 mm; and the balloon was left still in a constant temperature bath at 37°C for 7 days as it is inflated. Change in the amount of the injected water was determined, as the weight of the catheter was monitored. The residual water content was calculated according to the following Formula:

$$\text{Residual water rate} = \text{Weight of residual water} / \text{Weight of water injected} \times 100.$$

[Table 3]

| Elapsed days[day] | | 0 | 1 | 4 | 6 | 7 |
|---|---|---|---|---|---|---|
| Example 10 | Residual ratio [%] | 100 | 99 | 98 | 98 | 97 |
| Comparative Example 4 | Residual ratio [%] | 100 | 79 | 39 | 24 | 19 |

(Acid resistance)

The samples of Example 10 and Comparative Example 4 were immersed in an artificial gastric juice (see Japanese Pharmacopoeia B-476) at 37°C as they are inflated and the period until bursting (up to 60 days) was evaluated.
[Table 4]

| | Endurance period [day] |
|---|---|
| Example 10 | 60 < |
| Comparative Example 4 | 7 |

It is thus possible to provide a resin composition for balloons superior in gas permeation resistance and hydrolysis resistance (acid resistance) and also in the balance of flexibility, mechanical strength and elastic recoverability and a balloon prepared by using the same. The balloon can be used favorably as a gastrostomy balloon catheter.

BRIEF DESCRIPTION OF THE DRAWINGS

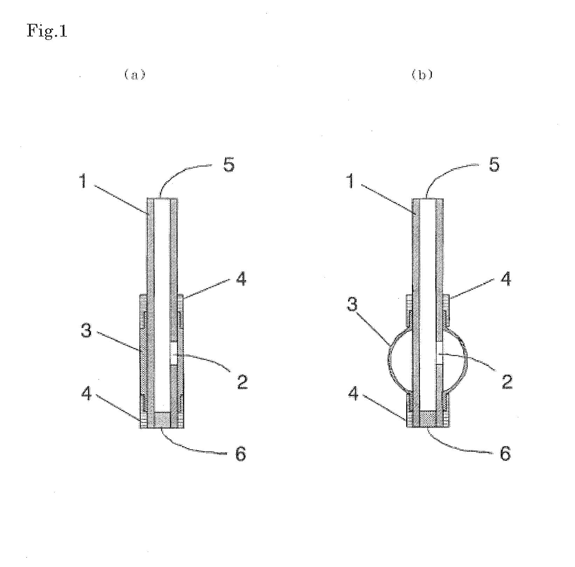

Figure 1 is a schematic crosssectional view illustrating the configuration of tube and the balloon during measurement of physical properties (inflation efficiency and internal pressure) of the balloon tube in Examples 7 to 9.
Figure (a) shows the state before inflation and Figure (b) shows after inflation.

EXPLANATION OF REFERENCES

1: Polytetrafluoroethylene tube
2: Hole
3: Tube for balloon
4: Shrink tube
5: Terminal of tube 1
6: Other terminal of tube 1

**Claims**

1. A resin composition for balloons, **characterized by** containing an isobutylene block copolymer (A) having a polymer block (a) mainly composed of isobutylene and a polymer block (b) mainly composed of an aromatic vinyl monomer, wherein the content of the polymer block (b) is 10 to 40% by weight of the isobutylene block copolymer (A).

2. The resin composition for balloons according to Claim 1, wherein the ratio (A)/(B) of the isobutylene block copolymer (A) to the thermoplastic polyurethane resin (B) is 100/0 to 40/60 by weight.

3. The resin composition for balloons according to Claim 1 or 2, wherein the molecular weight of the isobutylene block copolymer (A) is 50000 to 200000.

4. The resin composition for balloons according to any one of Claims 1 to 3, wherein the aromatic vinyl monomer is at least one monomer selected from the group consisting of styrene, p-methylstyrene and $\alpha$-methylstyrene.

5. The resin composition for balloons according to any one of Claims 1 to 4, wherein the isobutylene block copolymer (A) is a triblock copolymer having a polymer block mainly composed of an aromatic vinyl monomer, a polymer block mainly composed of isobutylene and a polymer block mainly composed of an aromatic vinyl monomer.

6. The resin composition for balloons according to any one of Claims 1 to 5, **characterized by** containing additionally a polymer (C) containing olefinic or styrenic polymers having at least one functional group selected from the group consisting of epoxy group, amino group, hydroxyl group, acid anhydride group, carboxyl group and the salts and carboxylic esters thereof in an amount of 0.1 to 50 wt parts with respect to 100 wt parts of the total amount of (A) and (B).

7. The resin composition for balloons according to any one of Claims 1 to 6, **characterized by** containing additionally a lubricant (D) in an amount of 0.1 to 10 wt parts with respect to 100 wt parts of the total amount of the isobutylene block copolymer (A) and the thermoplastic polyurethane resin (B).

8. The resin composition for balloons according to any one of Claims 1 to 7, **characterized by** containing additionally a processing aid (E) in an amount of 0.1 to 10 wt parts with respect to 100 wt parts of the total amount of the isobutylene block copolymer (A) and the thermoplastic polyurethane resin (B).

9. A balloon, **characterized by** being made of the resin composition for balloons according to any one of Claims 1 to 8.

10. A medical balloon, **characterized by** being made of the resin composition for balloons according to any one of Claims 1 to 8.

11. A gastrostomy balloon catheter, **characterized by** having a balloon made of the resin composition for balloons according to any one of Claims 1 to 8 in the distal region of a shaft having a lumen for flow of fluid for balloon inflation.

12. The gastrostomy balloon catheter according to Claim 11, wherein the water vapor permeance of the balloon is $1.06 \times 10^{-11}$ or less.

Fig.1

(a)                                        (b)

## INTERNATIONAL SEARCH REPORT

| | International application No. |
|---|---|
| | PCT/JP2008/069425 |

A. CLASSIFICATION OF SUBJECT MATTER
*C08L53/00*(2006.01)i, *A61L29/00*(2006.01)i, *C08L75/04*(2006.01)i

According to International Patent Classification (IPC) or to both national classification and IPC

B. FIELDS SEARCHED

Minimum documentation searched (classification system followed by classification symbols)
C08L53/00, A61L29/00, C08L75/04

Documentation searched other than minimum documentation to the extent that such documents are included in the fields searched
Jitsuyo Shinan Koho      1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,X | WO 2007/139199 A1 (Kaneka Corp.),<br>06 December, 2007 (06.12.07),<br>Full text<br>(Family: none) | 1-12 |
| X<br>Y | JP 2000-157627 A (Kuraray Co., Ltd.),<br>13 June, 2000 (13.06.00),<br>Claims; Par. Nos. [0004] to [0006], [0013] to<br>[0030], [0035], [0036], [0057]<br>(Family: none) | 1,3-12<br>2 |
| Y | JP 2000-44788 A (Teijin Ltd.),<br>15 February, 2000 (15.02.00),<br>Claims; Par. No. [0002]<br>(Family: none) | 2 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>13 November, 2008 (13.11.08) | Date of mailing of the international search report<br>22 December, 2008 (22.12.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2008/069425 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | WO 93/07217 A1 (Otsuka Pharmaceutical Factory, Inc.), 15 April, 1993 (15.04.93), Claims & US 5762944 A          & GB 2266892 A & GB 2266892 B          & GB 9311001 D & EP 559911 A1          & DE 4293170 T & CH 683673 A          & SE 9301719 A | 2 |
| A | JP 5-212104 A (Nippon Zeon Co., Ltd.), 24 August, 1993 (24.08.93), Claims; Par. Nos. [0007], [0019] (Family: none) | 1-12 |
| A | JP 5-310868 A (Japan Synthetic Rubber Co., Ltd.), 22 November, 1993 (22.11.93), Claims; Par. Nos. [0004] to [0019], [0028] (Family: none) | 1-12 |
| A | JP 6-172638 A (Becton Dickinson and Co.), 21 June, 1994 (21.06.94), Full text & DE 69317024 C          & AU 4467293 A | 1-12 |
| A | JP 2002-11092 A (Terumo Corp.), 15 January, 2002 (15.01.02), Claims; Par. Nos. [0001], [0002], [0014] to [0021] & US 2002/0002363 A1     & EP 1149598 A2 | 1-12 |

Form PCT/ISA/210 (continuation of second sheet) (April 2007)

**EP 2 204 416 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6218037 A